(19) Europäisches Patentamt
European Patent Office
Office européen des brevets

(11) Publication number: 0 025 351 A1

# (12) EUROPEAN PATENT APPLICATION

(21) Application number: 80303133.5

(22) Date of filing: 05.09.80

(51) Int. Cl.$^3$: C 12 Q 1/04
C 12 Q 1/66

(30) Priority: 05.09.79 GB 7930857

(43) Date of publication of application: 18.03.81 Bulletin 81/11

(84) Designated Contracting States: AT BE CH DE FR GB IT LI LU NL SE

(71) Applicant: DYNATECH AG
Gartenstrasse 2
CH-6300 Zug(CH)

(72) Inventor: Holley, John Ernest Foster
9 Shipfield Close Tatsfield
Westerham Kent(GB)

(74) Representative: Hose, Cyril Gustav Bidwell
Baron & Warren 16 Kensington Square
London W8 5HL(GB)

## (54) Detection of bacteria in urine.

(57) Preliminary testing of urine for the presence of bacteria is effected by (a) adding to a sample of urine an enzyme which leaches ATP from somatic cells present therein but not from any bacterial cells, (b) adding thereto a soluble magnesium salt and an enzyme which reacts with ATP with emission of light and recording the light emitted from the resulting solution, (c) when emission of light has ceased adding an enzyme which leaches ATP from any bacterial cells present therein and allowing to stand whilst leaching takes place, (d) repeating step (b) on the thus treated sample, and (e) utilising the data obtained in steps (b) and (d) to provide an indication of any bacteria present in the sample.

EP 0 025 351 A1

"Detection of Bacteria in Urine"

This invention relates to the detection of bacteria in urine.

The qualitative and quantitative examination of urine is frequently an important requirement both in human and veterinery medicine. Urine of human beings and other mammals contains a number of substances in solution some of which are the end products of animal metabolism whilst others are related to the condition of a patient and may arise, for example, from the patient being in a reproductive cycle (presence of compounds of the cyclopentanoperhydrophenanthrene series, e.g., pregnandione), from the partial or total failure of an organ, such as the pancreas, (increased glucose content) or from the presence of invading bodies viz. bacteria which are eliminated from the patient in the urine. Inorganic salts such as the alkali metal chlorides are another important constituent of urine. It is important to be able readily to determine the presence of bacteria in urine in the presence of these other substances.

It is known that certain enzymes will, in the presence of adenosine triphosphate (ATP), magnesium ions and oxygen, emit light within the visible spectrum for a short period of time of the order of 120 seconds. The emission of light is accompanied by decarboxylation of the enzyme and liberation of carbon dioxide. This reaction can be used to demonstrate the presence of adenosine triphosphate in an aqueous solution. However information does not appear to be available on the effect of other substances present in urine, whether present singly or in admixture, upon the course of the reaction.

A typical enzyme mixture which is readily available and which undergoes the reaction first discussed is the mixture known as luciferin-luciferase, obtainable from the mascerated bodies of fire flies. This reacts with ATP in accordance with the following scheme.

$$\text{luciferin/luciferase} + \text{ATP} \xrightarrow{Mg^{++}} \text{luciferin/luciferase} - \text{ATP}$$

$$\xrightarrow{O_2} \text{Decarboxyluciferin} + \text{luciferase} + \text{AMP} + CO_2 + \text{visible light}$$

(AMP = Adenosine monophosphate).

Provided that the various substances present in urine do not prevent the leaching out of ATP through the cell walls of somatic and bacterial cells and further providing that the presence of these same substances does not prevent the reaction between the light-producing enzyme and ATP it should be possible to make use of these phenomena to indicate the presence of bacteria in urine and also, if required, to estimate the amount thereof.

It is accordingly an object of the invention to provide a method for the detection of bacteria in urine in which use is made of the ability of certain enzymes to emit light in the presence of ATP.

The present invention provides a method for the detection of bacteria in urine which comprises:-

(a) adding to a discrete quantity of urine which is to be tested an enzyme which leaches ATP from somatic cells but not from bacterial cells present therein;

(b) then adding a soluble magnesium salt and an enzyme which reacts with ATP with emission of light and recording the light emitted from the resulting solution;

(c) after said emission of light has ceased adding to the solution an enzyme which leaches ATP from any bacterial cells present therein and allowing

said solution to stand whilst leaching takes place;

(d) adding to the resulting solution a soluble magnesium salt and an enzyme which reacts with ATP with emission of light and recording the light emitted from the resulting solution; and

(e) utilising the data obtained on the emission of light in steps (b) and (d) to provide an indication of any bacteria present in the sample of urine under test.

In the method of the present invention it is necessary to take into account that the normal functioning of the animal body can result in the presence in urine of some ATP. Usually the concentrations are small but they must be taken into account otherwise an incorrect result will be obtained. Moreover somatic cells are normally present in urine and these can undergo lysis with leaching of ATP through the walls. These potential sources of light must be eliminated before the presence or absence of bacterial cells can be determined. Accordingly in the method of the present invention an enzyme is first added which will effect lysis of somatic cells with leaching of ATP without effecting lysis of any bacterial cells which may be present e.g. an NRS cell lysing agent. The non-bacterial cells are mainly serum cells and sperm cells. The light emitted from the ATP leached from these somatic cells can be readily determined.

After light arising from the leaching of somatic cells has ceased to be emitted the test sample has added thereto another enzyme which will effect lysis of any bacterial cells with leaching of ATP through the walls e.g. an NRB cell lysing agent. The system is then allowed to stand for a suitable period for lysis to occur with leaching of ATP e.g. 10 minutes and it then has added thereto an enzyme which reacts with the ATP

present with emission of light and the light emitted as a result of this second lysis is then determined. It is this emission of light which essentially provides an indication of the bacteria present in the sample of urine under test. When the amount of light is carefully measured it can provide a quantitative indication of the bacteria present.

The same enzyme which reacts with ATP with emission of light is conveniently used in both light emission steps. The enzyme luciferin-luciferase already referred to is conveniently used for this purpose but the use of other equivalent enzymes is within the scope of the invention.

The enzyme luciferin-luciferase is normally made available with the requisite quantity of watersoluble magnesium salt: in the alternative the necessary quantity of salt solution should be added to the sample under test. No external supply of oxygen is necessary: there is sufficient available in the materials used for the present purpose.

The light emitted in conducting the tests is conveniently measured by means of a photoelectric cell having a sufficiently high degree of sensitivity. Since it is envisaged that all tests will be carried out under standardised conditions it is contemplated that a number of specimens of urine can be tested simultaneously using a row of similarly mounted and equally sensitive photoelectric cells or equivalent devices each connected to an appropriate recording circuit. Such photoelectric cells are conveniently disposed beneath and equidistant from a plurality of transparent tubes within which the samples to be tested are placed. When placed within a suitably darkened environment both the strength and duration of the light emitted can be recorded in each case, care being taken to ensure that light from only one sample under test is incident upon each photoelectric cell. Each recording circuit may include a read out print

device.

As an alternative to the use of a plurality of photoelectric cells a photoelectric detector may be employed which sequentially scans the bottom or a side of each of a series of transparent tubes.

When it is desired to compare the values for the light emitted in steps (b) and (d) of the method the signal derived from the emitted light recorded may be fed to a device which will print out or display the data recorded. The data obtained in step (b) may be stored in the device and then printed out or displayed in juxtaposition with that obtained in step (d). Such a device may comprise a microprocessor. The use of such devices enables rapid ascertainment as to which of a plurality of samples contain bacteria.

In carrying out the method of the invention a number of similar vessels each containing a sample of urine which it is desired to test for the presence of bacteria are placed side by side in a rack and the rack is mounted adjoining a tray having a number of transparent wells. By means of a corresponding number of sterile loops equal quantities of urine, one from each sample, are introduced into corresponding wells in the tray. A predetermined quantity of NRS cell lysing agent is next introduced into each well containing urine. This lysing agent lyses somatic cells but not bacterial cells. The thus inseminated wells are then allowed to stand for 15 to 30 seconds in order to leach ATP from the somatic cells. The tray containing the wells is then transferred to a machine which is provided with means for introducing equal quantities of the light emitting enzyme luciferin-luciferase into each well containing a lysed sample of urine. Underlying each well in this machine is a sensitive photoelectric device adapted to receive light from one of the wells. At least this part of the machine should be

provided with a darkened environment so that the only light incident upon each of the cells is that emanating from one of the wells containing lysed urine. The electric response from each photoelectric device is separately fed to a device which prints out or displays an indication proportional to the light incident thereon or stores it. When the light output has been recorded the tray is removed from the machine.

The next step is to add an equal quantity of NRB cell lysing agent to each of the wells containing already treated urine. The tray containing the wells is then allowed to stand for 5 to 10 minutes after which it is returned to the machine from which it was previously taken and a second equal quantity of the light-emitting enzyme luciferin-luciferase is introduced into each well containing a lysed sample of urine. The NRB cell lysing agent leaches ATP through the bacterial cell walls in each sample and when these are present ATP is liberated and is available in the solution for reaction with the light-emitting enzyme. Any light emitted is then determined and recorded in the same way as after lysing the somatic cells.

With respect to the volumes of urine and reagents used in the above procedure these can be very small. Thus 25 or 50 microlitres of urine may be used for each test and equal volumes of NRS and NRB cell lysing agents added thereto in the appropriate steps.

The data obtained and recorded in this way provide indications of both the amounts of somatic and bacterial cells present in the urine to be determined. Moreover if any substance is present which influences the reaction with the light emitting enzyme both readings for any given sample will be influenced in the same way and comparison of the readings will still enable a valid conclusion to be drawn.

When this procedure is adopted only those samples which reveal abnormalities in the somatic and/or bacterial cells present therein require further testing by conventional methods. In general this means that less than one-fifth, and often not more than one-tenth, of the samples will require to be submitted to the conventional testing methods with a corresponding saving in expense.

CLAIMS

1. A method for the detection of the presence of bacteria in urine characterized in that it comprises the following steps in the order given (a) adding to a discrete quantity of urine under test an enzyme which leaches ATP from somatic cells but not from bacterial cells present therein, (b) then adding a soluble magnesium salt and an enzyme which reacts with ATP with emission of light and recording the light emitted from the resulting solution, (c) after said emission of light has ceased from said solution adding an enzyme which leaches ATP from any bacterial cells present therein and allowing to stand whilst leaching takes place, (d) then adding thereto a soluble magnesium salt and an enzyme which reacts with ATP with emission of light and recording the light emitted from the resulting solution, and (e) utilizing the data obtained on the emission of light in steps (b) and (d) to provide an indication of any bacteria present in the sample of urine under test.

2. The method of claim 1 further characterized in that the same enzyme which reacts with ATP with emission of light is used in steps (b) and (d).

3. The method of either of claims 1 or 2 further characterized in that the enzyme which reacts with ATP with emission of light is luciferin-luciferase.

4. The method of any of claims 1 to 3 further characterized in that a plurality of samples of urine which have been introduced into a corresponding plurality of wells are simultaneously submitted to steps (a) to (e) of the method, the light emitted in steps (b) and (d) being used to transmit signals to a device which is adapted to provide a readout form thereof when required.

0025351

European Patent Office

# EUROPEAN SEARCH REPORT

Application number
EP 80 30 3133

| Category | Citation of document with indication, where appropriate, of relevant passages | Relevant to claim | CLASSIFICATION OF THE APPLICATION (Int. Cl.3) |
|---|---|---|---|
| | DE - A - 2 804 117 (CH.J. PLAKAS)<br>* Pages 26 and 27; example 6; page 25; example 4; page 22, line 7 - page 24, line 7 *<br>-- | 1,3 | C 12 Q 1/04<br>1/66 |
| | DE - A - 2 823 916 (S. KOLEHMAINEN et al.)<br>* Pages 2-4; claims 4 and 5; page 7, line 5-16 *<br>-- | 1-3 | |
| | US - A - 3 933 592 (J.R. CLENDENNING)<br>* Column 5, lines 42-53; column 10, claims 1-9 *<br>-- | 1,3 | TECHNICAL FIELDS SEARCHED (Int. Cl.3)<br>C 12 Q 1/04<br>1/06<br>1/66 |
| | CHEMICAL ABSTRACTS, vol. 82, no. 25, June 23, 1975, abstract 166972m, page 223<br>Columbus, Ohio, US<br>A. THORE et al.:"Detection of bacteriuria by luciferase assay of adenosine triphosphate"<br>& J. Clin. Microbiol. 1975, 1(1), 1-8<br>* Abstract *<br>-- | 1,3 | |
| | CHEMICAL ABSTRACTS, vol. 86, no.25 June 20, 1975, abstract 185399d page 245<br>Columbus, Ohio, US<br>G.L. PICCIOLO et al.: "Problem areas in the use of the firefly luciferase assay for bacterial detection"<br>./. | 1,3 | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons<br>&: member of the same patent family, corresponding document |

X The present search report has been drawn up for all claims

| Place of search | Date of completion of the search | Examiner |
|---|---|---|
| The Hague | 09-12-1980 | WALLINDER |

EPO Form 1503.1 06.78

European Patent Office

# EUROPEAN SEARCH REPORT

0025351

Application number

EP 80 30 3133

-2-

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| | & NASA Spec. Publ. 1975, NASA SP-388, Anal. Appl. Biolumin. Chemilumin, 1-26<br>* Abstract *<br>-- | | |
| A | A.L. LEHNINGER: "Biochemistry" 1970, Worth Publishers, Inc. Part 1: The molecular components of cells, page 234 New York US<br>* Page 234, last paragraph *<br>-- | 1c | |
| | | | TECHNICAL FIELDS SEARCHED (Int. Cl. 3) |
| A | CHEMICAL ABSTRACTS, vol. 86, no.3 January 17, 1977, abstract 12216p page 63 Columbus, Ohio, US O.V. BUKHARIN et al.: "Characteristics of lysozyme antimicrobial activity"<br>& Antibiotiki (Moscow) 1976, 21(9), 805-8<br>* Abstract *<br>-- | 1c | |
| A | CHEMICAL ABSTRACTS, vol. 82, no. 23, June 9, 1975, abstract 152070d page 273 Columbus, Ohio, US I.A. CHERKASOV et al.: "Bacteriolytic effect of lysozyme"<br>& Biokhimiya 1974 39(6), 1308-11<br>* Abstract *<br>---- | 1c | |